# EUROPEAN PATENT SPECIFICATION

(11) **EP 3 327 726 B1**
(45) Date of publication and mention of the grant of the patent: **26.06.2024**
(21) Application number: 17199835.4
(22) Date of filing: 03.11.2017
(51) Int. Cl.: G16H 30/20, G06N 3/045, G06N 3/08, G06N 7/01, G06N 20/10

(54) **ANONYMOUS AND SECURE CLASSIFICATION USING A DEEP LEARNING NETWORK**
ANONYME UND SICHERE KLASSIFIZIERUNG MITHILFE EINES DEEP-LEARNING-NETZWERKS
CLASSIFICATION ANONYME ET SÉCURISÉE UTILISANT UN RÉSEAU D'APPRENTISSAGE PROFOND

(30) Priority: 04.11.2016 US 201615344321
(43) Date of publication of application: 30.05.2018
(73) Proprietor: Siemens Healthineers AG, 91301 Forchheim (DE)
(72) Inventor: Kiraly, Atilla Peter, San Jose, CA 95128 (US); Gall, Peter, 91080 Uttenreuth (DE)
(74) Representative: Bals & Vogel Patentanwälte PartGmbB

(56) References cited:
- WO-A1-2015/188275
- CN-A- 105 868 678
- CN-A- 105 868 678
- Jialan Que ET AL: "A collaborative framework for Distributed Privacy-Preserving Support Vector Machine learning", AMIA ... Annual Symposium proceedings / AMIA Symposium. AMIA Symposium, 1 January 2012 (2012-01-01), page 1350, XP055461727, United States Retrieved from the Internet: URL:https://www.ncbi.nlm.nih.gov/pmc/artic les/PMC3540462/pdf/amia_2012_symp_1350.pdf
- MART\'IN ABADI ET AL: "TensorFlow: Large-Scale Machine Learning on Heterogeneous Distributed Systems", ARXIV.ORG, CORNELL UNIVERSITY LIBRARY, 201 OLIN LIBRARY CORNELL UNIVERSITY ITHACA, NY 14853, 14 March 2016 (2016-03-14), XP080689634,
- H. Brendan Mcmahan ET AL: "Communication-Efficient Learning of Deep Networks from Decentralized Data", , 17 February 2016 (2016-02-17), XP055467287, Retrieved from the Internet: URL:https://arxiv.org/pdf/1602.05629v2.pdf
- LU YUNMEI ET AL: "Privacy Aware Non-linear Support Vector Machine for Multi-source Big Data", 2014 IEEE 13TH INTERNATIONAL CONFERENCE ON TRUST, SECURITY AND PRIVACY IN COMPUTING AND COMMUNICATIONS, IEEE, 24 September 2014 (2014-09-24), pages 783-789, XP032724996, DOI: 10.1109/TRUSTCOM.2014.103 [retrieved on 2015-01-15]
- Bargav Jayaraman: "Privacy Preserving Machine Learning: Related Work", , 6 December 2015 (2015-12-06), XP055467251, Retrieved from the Internet: URL:https://pdfs.semanticscholar.org/c672/ c89d5899949ef0662046b65ea0d8e8771fd4.pdf
- Marco A Wiering ET AL: "Multi-Layer Support Vector Machines", Regularization, Optimization, Kernels, and Support Vector Machines, 1 January 2013 (2013-01-01), XP055351511, Retrieved from the Internet: URL:http://www.ai.rug.nl/~mwiering/GROUP/A RTICLES/Multi-Layer-SVM.pdf [retrieved on 2017-03-03]
- MATT FREDRIKSON ET AL: "Model Inversion Attacks that Exploit Confidence Information and Basic Countermeasures", PROCEEDINGS OF THE 22ND ACM SIGSAC CONFERENCE ON COMPUTER AND COMMUNICATIONS SECURITY, CCS '15, 1 January 2015 (2015-01-01), pages 1322-1333, XP055468313, New York, New York, USA DOI: 10.1145/2810103.2813677 ISBN: 978-1-4503-3832-5

## Description

### BACKGROUND

The present embodiments relate to machine learning applied by a remote server. Cloud-based processing may apply the latest machine learning methods and application features to provide rapid and robust results for a variety of purposes. For example, face detection and labeling are done on social networking sites via cloud-based applications. In cases where privacy is a concern, such as medical data, the same approach may be problematic due to compliance rules (e.g., as Health Insurance Portability and Accountability Act (HIPPA)). Additionally, in some cases, people prefer privacy when dealing with personal photos. Military applications also require substantial security if processing is done offsite. Finally, as data has value, the owner of the data may not be willing to share the data in the cloud despite the advantages, but may instead prefer to only locally run the application.

Removing identifiers (anonymization) and encrypting data for transmission and storage is one solution for allowing private cloud-based applications. The owner of the data must completely trust the application provider in properly handling, encrypting, deleting, and using the data. However, encryption and servers may be breached, and therefore may not provide sufficient protection of privacy. Even with anonymization, the data can be used to identify the patient. In the case of photographs, the person can easily be identified if the data is compromised. In morphological medical images, the patient can be identified by shape features. To avoid these concerns, the application may be run locally on the user's machine. This situation surrenders the ability of having the latest application and cloud-based processing advantages. Such systems are typical trained with a database of labeled images or volumes, and, once deployed, remain fixed in terms of learning from new data. Unfortunately, in this situation, the system may never learn from mistakes made while in clinical use. Additionally, such local systems occupy space and must frequently be run on specialized workstations. Relevant prior art can be found in:
JIALAN QUE ET AL.: A collaborative framework for Distributed Privacy-Preserving Support Vector Machine learning. AMIA Annual Symposium proceedings / AMIA Symposium. 1 January 2012, page 1350;
MARTIN ABADI ET AL.: TensorFlow: Large-Scale Machine Learning on Heterogeneous Distributed Systems. ARXIV.ORG, CORNELL UNIVERSITY LIBRARY, 201 OLINE LIBRARY CORNELL UNIVERSITY ITHACA, NY 14853, 14 March 2016;
H. BRENDAN MCMAHAN ET AL.: Communication-Efficient Learning of Deep Networks from Decentralized Data. 17 February 2016;
LU YUNMEI ET AL.: Privacy Aware Non-linear Support Vector Machine for Multi-source Big Data. 2014 IEEE 13TH INTERNATIONAL CONFERENCE ON TRUST, SECURITY AND PRIVACY IN COMPUTING AND COMMUNICATIONS, IEEE, 24 September 2014, pages 783-789;
WO 2015/188275 A1;
CN 105868678 A;
BARGAV JAYARAMAN: Privacy Preserving Machine Learning: Related Work. 6 December 2015;
MARCO A. WIERING ET AL.: Multi-Layer Support Vector Machines. Regularization, Optimization, Kernels, and Support Vector Machines, 1 January 2013;
MATT FREDRIKSON ET AL.: Model Inversion Attacks that Exploit Confidence Information and Basic Countermeasures. PROCEEDINGS OF THE 22ND ACM SIGSAC CONFERENCE ON COMPUTER AND COMMUNICATIONS SECURITY, CCS '15, 1 January 2015, pages 1322-1333;

### BRIEF SUMMARY

By way of introduction, the preferred embodiments described below include methods, systems, instructions, and non-transitory computer readable media for use of machine-learnt classifier for anonymous data transfer. Deep learning, such as neural network machine learning, results in a classifier with multiple distinct layers. Each layer processes the output of a preceding layer. As compared to the input to the layer, the output is different. By applying a subset of layers locally, the resulting output is provided to a cloud server for application to the remaining layers. Since the output of a layer of the deep-learnt classifier is different than the input, the information transmitted to and available at the cloud server is more anonymous or different than the original data, yet the cloud server may apply the latest machine learnt classifier as the remaining layers.

In a first aspect, a method is provided for use of machine-learnt classifier in medical imaging. A medical scanner acquires scan data representing a patient. A first processor processes the scan data through a first set of layers of a deep-learnt network. The deep-learnt network includes the first set of layers and a second set of layers, and the first set of layers includes two or more layers. An output of the first set of layers is transmitted over a communications network from the first processor to a second processor. The output is different than the scan data and is anonymous to the patient. The second processor processes the output of the first set of layers through the second set of layers of the deep-learnt network. An output of the second set of layers is a classification of the scan data. The classification of the scan data for the patient is transmitted over the communications network from the second processor to the first processor.

Preferred is a method, wherein acquiring scan data comprises acquiring the scan data as computed tomography, magnetic resonance, ultrasound, positron emission tomography, or single photon emission computed tomography data.

Preferred is further a method, wherein processing the scan data comprises processing with the deep-learnt network being a neural network learnt from training data of scans of other patients with known classifications,
and/or
wherein processing the scan data comprises processing with the first set of layers comprising a convolutional layer, a max pooling, or both the convolutional layer and the max pooling layers; and
wherein processing the output comprises processing with the second set of layers comprising a fully connected layer, an up convolutional layer, or both the fully connected layer and the up convolutional layer.

Processing the scan data can comprise inputting the scan data to a first layer of the first set of layers, and inputting an output of the first layer to a second layer of the first set of layers.

Preferred is a method, wherein transmitting the output comprises transmitting the output as abstracted data relative to the scan data, and wherein transmitting the classification comprises transmitting the classification as identification of anatomy, identification of a lesion, identification as benign or malignant, or staging.

Alternatively or additionally, a method is preferred, wherein transmitting the output comprises transmitting from the first processor in a facility with the medical scanner to the second processor, the second processor comprising a cloud server.

Processing the output can comprise processing with two or more layers in the second set of layers.

Preferred is further a method, wherein the method further can comprise:
- storing parameters of the first set of layers as encrypted,
and/or further can comprise:
- encrypting the output, wherein transmitting the output comprises transmitting the output as encrypted; and
- decrypting the output as encrypted by the second processor prior to processing the output.

The method further comprises:
- transmitting a physician verified result of the classification to the second processor; and
- retraining the second set of layers of the deep learnt network based on the physician verified result and the output of the first set of layers and without retraining the first set of layers.

In a second aspect, not falling under the scope of the invention, a method is provided for use of machine-learnt classifier for anonymous data transfer. The first n layers of a neural network comprised of a total of k layers is operated on a first computer in a first location. Activation data resulting from the first part of the neural network is transmitted to a cloud server at a second location remote from the first location. An output of the remaining k-n layers of the neural network is received by the first computer from the cloud server. This output is from operation of just a second part of the neural network. The output is displayed on the first computer.

Preferred is a method according the second aspect, wherein operating comprises operating on data for a person, and wherein transmitting comprises transmitting the activation data where the operating of the first part removes identifying information of the person.

Preferred is a method according the second aspect, further comprising encrypting parameters of the first part of the neural network as stored on the first computer,
Further, preferred is a method according the second aspect, further comprising transmitting a correct label for the output to the cloud server.

Preferred is a method according the second aspect, wherein operating comprises operating a medical imaging data of a patient, wherein transmitting comprises transmitting the activation data as different than the medical imaging data, and wherein receiving the output comprise receiving a classification of the medical imaging data,
and/or
wherein operating comprises operating on a photograph, wherein transmitting comprises transmitting the activation data as different than the photograph, and wherein receiving the output comprises labeling content of the photograph,
   and/or
wherein operating comprises operating on measurements of a person from a sensor, wherein transmitting comprises transmitting the activation data as compressed by the operating, and wherein receiving the output comprises receiving analysis of the measurements for the person.

The method according the second aspect can further comprise:
- transmitting a selection of an application to the cloud server;
wherein receiving the output comprises receiving the output based on the second part being selected based on the selection,
and/or can further comprise:
- transmitting a physician verified result of the output to the cloud server; and
- retraining the second part of the neural network based on the physician verified result and the output of the first part of the neural network and without retraining the first part.

In a third aspect, not falling under the scope of the invention, a method is provided for use of machine-learnt classifier for anonymous data transfer. Feature data anonymous to a person due to application of original data to part of but not all of machine-learnt processing is received from a first machine. A second machine performs a remainder of the machine-learnt processing with the feature data as input. Results of the machine-learnt processing are transmitted to the first machine.

The present invention is defined by the following claims, and nothing in this section should be taken as a limitation on those claims. Further aspects and advantages of the invention are discussed below in conjunction with the preferred embodiments and may be later claimed independently or in combination.

### BRIEF DESCRIPTION OF THE DRAWINGS

The components and the figures are not necessarily to scale, emphasis instead being placed upon illustrating the principles of the invention. Moreover, in the figures, like reference numerals designate corresponding parts throughout the different views.
Figure 1 is a block diagram of one embodiment of a system for use of machine-learnt classifier for anonymous data transfer;
Figure 2 illustrates example distribution of different layers between local and cloud machines for anonymous data transfer and cloud-based application of a machine-learnt classifier;
Figure 3 illustrates one embodiment of a method for use of machine-learnt classifier in medical imaging;
Figure 4 is a flow chart diagram of one embodiment of a method for use of machine-learnt classifier for anonymous data transfer; and
Figure 5 illustrates example compression and anonymization provided by implementing one or more layers of a neural network locally.

### DETAILED DESCRIPTION OF THE DRAWINGS AND PRESENTLY PREFERRED EMBODIMENTS

Current applications either run locally or run in the cloud with potential privacy concerns. For cloud-based applications, the data is transferred to the cloud. The user must trust the application provider to not use the data for other purposes and to not allow access to the data by others. The network and encryption must be secure to maintain privacy. Providing information for retraining the application requires further trust. For applications run locally, the data is not transferred to the cloud, so there is less risk of privacy violation and no need to trust a cloud provider. Any upgrades are physically installed, so the latest version of the application may not be available. The local machine location, accessibility, and hardware may limit effectiveness and usage. Retraining may be limited to just samples available at the one location and may be difficult to manage where an expert for the application is not available at the local facility.

These trade-offs may be avoided with the approaches presented. Anonymized and secure cloud processing with or without incremental training is provided using deep learning networks. Neural network or other classifier parameters are provided by deep learning or other machine learning approaches. The first layer accepts and processes the raw data (e.g., pictures, medical image volumes, electrocardiogram data, etc.). This raw data may include private information or may be used to relate a given case to a person. The first layer and/or later layers progressively output higher level content of the original data relevant to the network's intended purpose. The first layers are performed locally. The data from the nth hidden layer is sent to the cloud to compute the final output using remaining layers. This data from the nth layer is inherently anonymous or more anonymous than the original data. For example, a network that is designed to identify faces will extract abstract face concepts relevant to face identification in the hidden layers. This same content will not be as useful for another task such as identifying cars in an image if the network is not trained for such cases. Similarly, a network trained to identify the sternum in a medical image may not contain the necessary features on the deeper level to be repurposed for another usage. This abstraction property is employed to provide data for further processing by a cloud-based application. By only passing data from deeper layers of the neural network, the original data is not put at risk by transfer, and re-usage for other purposes is further limited. The cloud server applies the received data from the deeper layers to the rest of the neural network, providing a result from the neural network.

By selecting the number of layers of the deep-learnt classifier to implement locally, a granularity in the factor of data security and reusability is provided. Each layer adds more abstraction as compared to the output of previous layers, so more layers run locally increases privacy or reduces risk. This guarantee may promote further data sharing and usage due to additional security and limited re-use guarantees, making more data available for retraining.

For retraining, the parts of the neural network implemented in the cloud may be retrained without altering the local parts of the neural network. For further protection, the parameters of the layers implemented locally may be encrypted. Even in a case of security violation providing access to the output to the cloud, the parameters are needed to reverse, if even possible, the transmitted data to derive the original data.

Truly anonymized data is transferred in cloud-based applications where the data may also be used to incrementally train the deep learning network for improved results. The possibility of using the data for other purposes than intended may be reduced. Substantial trust of the application provider is no longer as necessary with this solution as breaching the cloud server does not violate the privacy. The cloud server does not have the identification information, and the information possessed is not easily used to identify. The data sent to the cloud server is used only for the improvement of the current application and cannot be used for other purposes. The data is private by design.

The machine-learnt network may have one of various applications. The classifier is used for medical environments, such as to classify tissue or lesions, evaluation for prognosis or diagnosis, or to produce segmentations. Alternatively, not falling under the scope of the invention, the classifier is for person recognition, object recognition, or other analysis in or not in a medical environment. The discussion below uses a medical environment where the identity of the patient is to be kept secure, but other environments may be used where the data is kept secure.

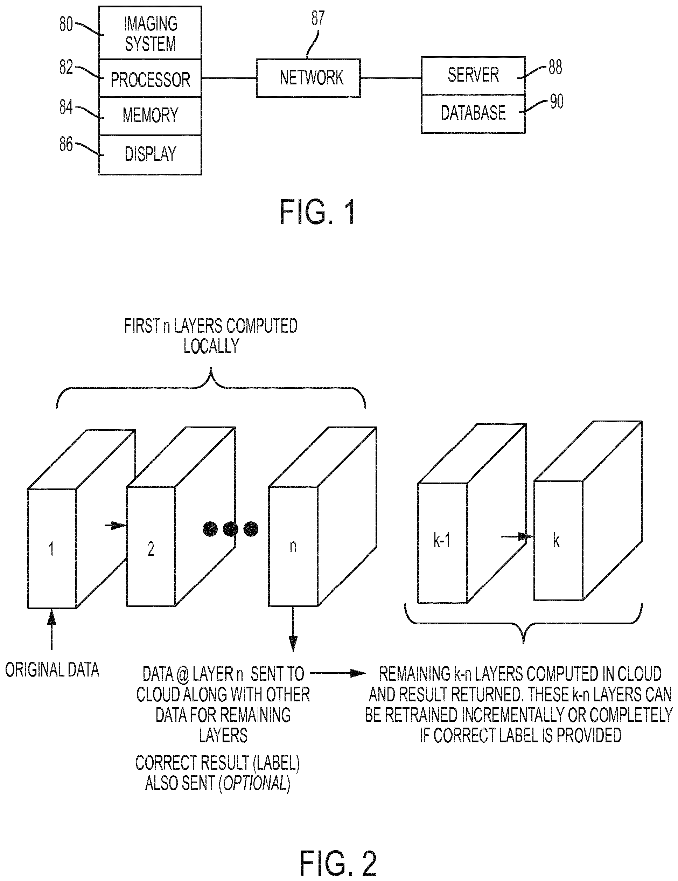

Figure 1 is a block diagram of one embodiment of a system for use of machine learning with more anonymous data transfer than transfer of the original data. The system includes an imaging system 80, a processor 82, a memory 84, a display 86, a communications network 87, a server 88, and a database 90. Additional, different, or fewer components may be provided. For example, network connections or interfaces are provided, such as for networking with a medical imaging network or data archival system. In another example, a user interface is provided. As another example, the server 88 and database 90 are not provided, or only the server 88 and database 90 are provided. In other examples, the server 88 connects through the network 87 with many imaging systems 80 and/or processors 82.

The processor 82, memory 84, and display 86 are part of the medical imaging system 80. Alternatively, the processor 82, memory 84, and display 86 are part of an archival and/or image processing system, such as associated with a medical records database workstation or server, separate from the imaging system 80. In other embodiments, the processor 82, memory 84, and display 86 are a personal computer, such as desktop or laptop, a workstation, a server, a network, or combinations thereof. The processor 82, display 86, and memory 84 may be provided without other components for acquiring data by scanning a patient.

The imaging system 80, processor 82, memory 84 and display 86 are provided at a same location. The location may be a same room, same building, or same facility. This location is local relative to the server 88. The server 88 is spaced apart by the network 87 by being in a different facility or by being in a different city, county, state, or country. The server 88 and database 90 are remote from the location of the processor 82 and/or imaging system 80.

The imaging system 80 is a medical diagnostic imaging system. Ultrasound, computed tomography (CT), x-ray, fluoroscopy, positron emission tomography (PET), single photon emission computed tomography (SPECT), and/or magnetic resonance (MR) systems may be used. The imaging system 80 may include a transmitter and includes a detector for scanning or receiving data representative of the interior of the patient.

In one embodiment, the imaging system 80 is a CT system. An x-ray source is connected with a gantry. A detector is also connected with a gantry opposite the x-ray source. The patient is positioned between the source and detector. The source and detector are on opposite sides of the patient and rotate and/or translate about the patient. The detected x-ray energy passing through the patient is converted, reconstructed or transformed into data representing different spatial locations within the patient.

In another embodiment, the imaging system 80 is a MR system. The MR system includes a main field magnet, such as a cryomagnet, and gradient coils. A whole body coil is provided for transmitting and/or receiving. Local coils may be used, such as for receiving electromagnetic energy emitted by atoms in response to pulses. Other processing components may be provided, such as for planning and generating transmit pulses for the coils based on the sequence and for receiving and processing the received k-space data. The received k-space data is converted into object or image space data with Fourier processing.

The memory 84 may be a graphics processing memory, a video random access memory, a random access memory, system memory, cache memory, hard drive, optical media, magnetic media, flash drive, buffer, database, combinations thereof, or other now known or later developed memory device for storing data or video information. The memory 84 is part of the imaging system 80, part of a computer associated with the processor 82, part of a database, part of another system, a picture archival memory, or a standalone device.

The memory 84 stores medical imaging data representing the patient, weights or values of parameters making up some of the layers of the machine-learnt classifier, outputs from different layers, a portion of a machine-learnt matrix, and/or images. The memory 84 may alternatively or additionally store data during processing.

The memory 84 or other memory is alternatively or additionally a non-transitory computer readable storage medium storing data representing instructions executable by the programmed processor 82 for use of a machine-learnt classifier in medical imaging. The instructions for implementing the processes, methods and/or techniques discussed herein are provided on non-transitory computer-readable storage media or memories, such as a cache, buffer, RAM, removable media, hard drive or other computer readable storage media. Non-transitory computer readable storage media include various types of volatile and nonvolatile storage media. The functions, acts or tasks illustrated in the figures or described herein are executed in response to one or more sets of instructions stored in or on computer readable storage media. The functions, acts or tasks are independent of the particular type of instructions set, storage media, processor or processing strategy and may be performed by software, hardware, integrated circuits, firmware, micro code and the like, operating alone, or in combination. Likewise, processing strategies may include multiprocessing, multitasking, parallel processing, and the like.

In one embodiment, the instructions are stored on a removable media device for reading by local or remote systems. In other embodiments, the instructions are stored in a remote location for transfer through a computer network or over telephone lines. In yet other embodiments, the instructions are stored within a given computer, CPU, GPU, or system.

The processor 82 is a general processor, central processing unit, control processor, graphics processor, digital signal processor, three-dimensional rendering processor, image processor, application specific integrated circuit, field programmable gate array, digital circuit, analog circuit, combinations thereof, or other now known or later developed device for machine-learnt classification. The processor 82 is a single device or multiple devices operating in serial, parallel, or separately. The processor 82 may be a main processor of a computer, such as a laptop or desktop computer, or may be a processor for handling some tasks in a larger system, such as in the imaging system 80. The processor 82 is configured by instructions, design, hardware, and/or software to perform the acts discussed herein.

The processor 82 is configured to perform the acts discussed above for the local machine. In one embodiment, the processor 82 is configured to implement part, but not all, of the machine-trained classifier. A subset of layers is implemented. The original data is input to the first layer. Any number of additionally layers are implemented in sequence, resulting in abstraction of the original data. This abstracted data is more anonymous such that the data is different than the original data in a way that viewing the data in the appropriate format (e.g., image, medical record, or table) cannot be used to identify the patient.

The processor 82 is configured to transmit the abstracted data to the server 88 over the network 87 and receive classification results from the server 88. The processor 82 may be configured to generate a user interface for receiving corrections or verification of classification results and provide the correction or verification to the server 88.

The display 86 is a monitor, LCD, projector, plasma display, CRT, printer, or other now known or later developed devise for outputting visual information. The display 86 receives images, graphics, text, quantities, or other information from the processor 82, memory 84, imaging system 80, and/or server 88. One or more medical images are displayed. The images are of a region of the patient. The image includes an indication, such as a graphic or colorization, of the classification results, such as located anatomical structure or an annotation of a type of lesion (e.g., benign or malignant). Alternatively or additionally, the image includes a quantity based on the classification. The quantity may be displayed as the image without a medical image representation of the patient. In other embodiments, the classification results are displayed without a medical image representing the anatomy of the patient.

The network 87 is a local area, wide area, enterprise, another network, or combinations thereof. In one embodiment, the network 87 is, at least in part, the Internet. Using TCP/IP communications, the network 87 provides for communication between the processor 82 and the server 88. Any format for communications may be used. In other embodiments, dedicated or direct communication is used.

The server 88 is a processor or group of processors. More than one server 88 may be provided. The server 88 is configured by hardware and/or software to receive the abstracted data output by the final layer in the subset of layers implemented by the processor 82. The server 88 uses this abstracted data to classify. The remaining layers of the machine-learnt classifier are applied with the abstracted data as input to a first one of the remaining layers. The last of the remaining layers outputs the classification results.

The server 88 may be further configured to create a database of abstracted data and corresponding classifications. Feedback about correctness of the classification may be provided. This feedback may be used for retraining. Alternatively, abstracted data and verified classification without reference to a classification output by the machine-learnt classifier are stored for retraining.

The database 90 is a memory, such as a bank of memories, for storing abstracted data, weights or values of parameters of the remaining layers implemented by the server 88, a matrix representing the remaining part of the classifier, and/or respective classifications. For retraining, the database 90 stores the corrected classifications and/or physician verified classifications for each set of abstracted data.

The machine-learnt classifier is created with training data. The machine-learnt classifier classifies tissue, the patient, or other data. The classification is an identification, a segmentation (e.g., classify where an organ is located), a prognosis, a diagnosis, or other characterization of the data. Samples of input data with ground truth are used to learn to classify, evaluate, or segment by the machine-learnt system based on the input data. The classifier learns the features of the input data to extract from the training data. Alternatively, the features, at least for the input, are manually programmed, such as filtering the scan data and inputting the results of the filtering. The training relates the input data to the classification through one or more layers. One layer may be the creation of a feature set to be input. For deep-learnt networks, there may be further layers creating further abstract features from outputs of pervious layers. The resulting machine-trained classifier is a matrix for inputs, weighting, and combination to output a classification and/or probability of class membership. The machine-trained classifier includes two or more layers relating the input to the class.

Any machine learning or training may be used. In one embodiment, a neural network is used. Other deep learnt, sparse auto-encoding classifiers may be trained and applied. The machine training is unsupervised in learning the features to use and how to classify given a feature vector. In alternative embodiments, a Bayes network or support vector machine are trained and applied. Hierarchal or other approaches may be used. Supervised or semi-supervised machine learning may be used.

After creation, the machine-learnt classifier includes at least two layers. For a manually programmed features, the two layers may be feature calculation in one layer and a network relating the features to the class in another layer. For a deep-learnt network, at least the feature layer is learned from the training data rather than manually programmed. More than two layers may be provided, such as a neural network with three or more layers.

The machine-trained classifier is distributed between the processor 82 and the server 88. Since the processor 82 implements some of the machine-trained classifier, the data output by the processor 82 to the server 88 is more anonymous. The abstraction changes the nature of the data into a form specific to the machine-learnt classifier, so that the output from the hidden layer has limited use and may not be used to identify the patient.

The layers are divided between local processing and cloud processing. Figure 2 shows distribution of k layers of a machine-learnt classifier distributed between local and remote or cloud processing. A partial cloud-based deep neural network is provided due to the distribution. As different layers process the information from the previous layer, the information becomes more abstract and sometimes compressed. Given a network of a total of k layers, where each layer is one of a fully connected, convolutional, max pooling, up convolution, or other layer, the data up to the nth layer is computed locally by the processor 82. The output of the nth layer is sent to the cloud server 88. At the same time, a later time, or never, a correct label for the classification is also sent. The remaining k-n layers are applied at the cloud server 88. The server 88 returns the results to the processor 82 or to another location or computer.

By deciding which layer n to output data for sending for further processing, a granularity allows for a custom balance between information abstraction and the original data. For retraining, the correct label given to the cloud application is used to re-train (incrementally or fully with previous data) the final k-n layers of the network to provide improved results in the future. The choice of n decides what portion of the network may be retrained versus the amount of abstraction of the original data. Setting n=0 results in a standard cloud-based application where the bare data is sent and may be used for any purpose without a mechanism to guarantee off-label use to the user. Higher values of n result in more abstract data and more "frozen" layers (1-n) that cannot be retrained without re-deployment or updates to the local machine.

By sending the activation only at a layer n≥1, the local application is in fact sending only features of interest in the data, not the entire dataset. Also, depending upon the inputs of layer n+1, the data may also be compressed. Additionally, since only the first n layers exist on the local device, the local system cannot be reversed engineered to extract the complete neural network as would be possible on a stand-alone machine running the entire neural network.

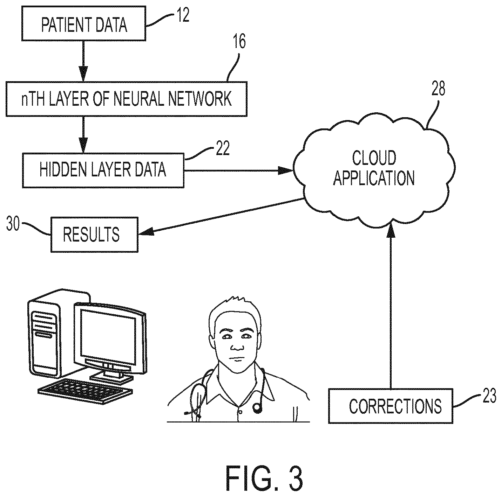

Figure 3 shows one embodiment of a method for use of a machine-learnt classifier for more anonymous data transfer. The method is illustrated in the context of medical imaging, but may be applied in other contexts. The machine-learnt classifier is separated sequentially, so that a first part is performed locally and final part is performed in the cloud (see Figure 2). The first part is used to alter the data to be transmitted. The transmitted data used at the cloud server is more anonymous than original data.

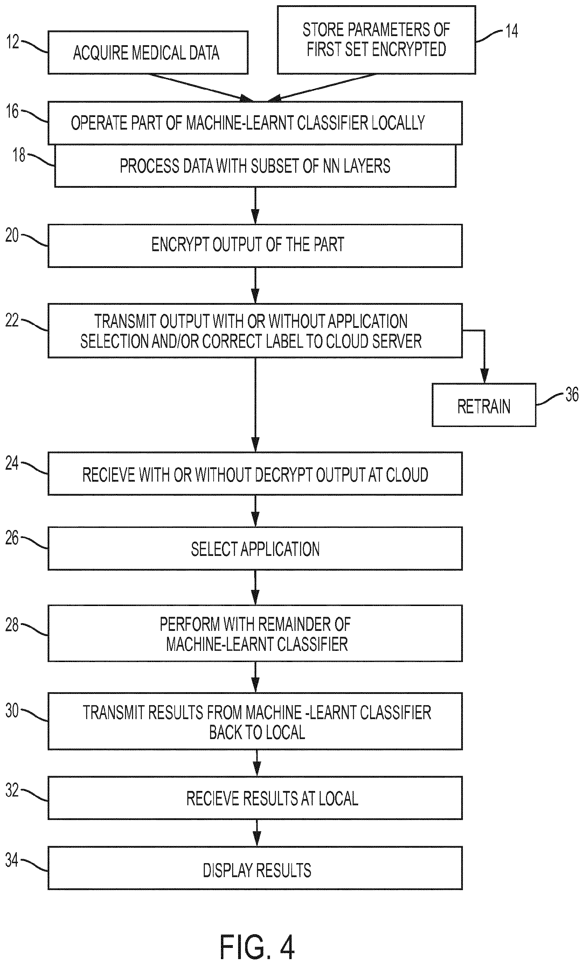

Additional, different, or fewer acts may be provided. For example, Figure 4 is another embodiment of the method. Figure 4 provides a more detailed example of the method of Figure 3 with additional acts. As another example, act 36 is not performed. For Figure 4, any of various acts may not be provided, such as acts 14, 20, 26, and/or 36.

The methods of Figures 3 and 4 are performed by the system of Figure 1 or other systems. For example, acts 12-22 and 32-34 are performed by a local machine, such as a computer or imaging system at a hospital or other facility, and acts 24-30 and 36 are performed in the cloud, such as by a cloud server remote from the local machine.

The acts are performed in the order shown (e.g., top to bottom) or other orders. For example, act 14 is performed prior to act 12. As another example, act 22 is separated out into different transmissions for different types of data with the correct label being provided after act 34 such that act 36 is also then performed after act 34. Alternatively, act 36 is performed prior to any of acts 26-34.

In Figure 3, patient data is acquired in act 12. Locally, a machine runs a partial neural network of n layers in act 16. The activation data from the nth or last layer of the partial network is abstracted from the original patient data. This activation data is hidden layer data of the neural network and is sent in act 22 to the cloud for continued processing in act 28 via the remainder of the neural network. In such a solution, the institution (i.e., entity at the local facility) retains the original patient data and only the anonymized feature data, which has no clear resemblance the original patient images, are passed in act 22. Since the data being passed is comprised solely of features idealized for this particular application, the user or hospital is more likely to share and send the data since the value of the data is mainly for this particular purpose. In act 30, the results from application of the complete neural network are provided back to the local facility, such as back to the same machine that implemented act 16. The results are displayed for diagnosis or prognosis.

Both the hidden layer data and the correct label are sent for retraining the cloud based k-n layers in optional act 36. The physicians (users) at the local facility or multiple local facilities using the cloud server correct or affirm the given results. These corrections and affirmations may be used for improving the application. Once any number of additional samples of corrected labels or classification are collected, the algorithm may be machine trained with the given data on the k-n layers to provide improvements. New approaches to classification and/or learning may be implemented for the k-n layers implemented by the cloud server. Additional information, such as some original data or data output by a layer before layer n, may be used by layers in k-n depending upon the architecture of the neural network. This additional information may be sent as well with the understanding that the additional information may reduce anonymity.

Referring to Figure 4, medical data is acquired in act 12. A medical scanner, sensor, and/or user interface for manual entry of information acquires the data. A processor may extract the data from a picture archive communications system or a medical records database. Alternatively, data not in the medical environment is acquired, such as capturing or loading a photograph or video.

The medical image or dataset is acquired by an imaging system. Alternatively, the acquisition is from storage or memory, such as acquiring a previously created dataset from a PACS.

Any type of medical data may be acquired. For example, the data is family history, lab results, EKG, pulse-ox, clinical data, or other sensed data. In one embodiment, at least some of the data is acquired with a medical scanner. Any type of medical scanner and corresponding scan data representing the patient may be obtained. For example, CT, MR, ultrasound, PET, or SPECT scan data is obtained.

The scan data is medical imaging data. The medical image is a frame of data representing the patient. The data may be in any format. While the terms image and imaging are used, the image or imaging data may be in a format prior to actual display of the image. For example, the medical image may be a plurality of scalar values representing different locations in a Cartesian or polar coordinate format different than a display format. As another example, the medical image may be a plurality red, green, blue (e.g., RGB) values output to a display for generating the image in the display format. The medical image may be currently or previously displayed image in the display or another format. The image or imaging is a dataset that may be used for imaging, such as scan data representing the patient.

Any type of medical image may be used. In one embodiment, the medical image is a chest CT image acquired with a CT system. For example, a chest CT dataset may be used for detecting a bronchial tree, fissures, and/or vessels in the lung. For CT, the raw data is reconstructed into a three-dimensional representation. As another example, MR data representing a patient is acquired. MR data is acquired with an MR system. The data is acquired using an imaging sequence for scanning a patient. Data representing an interior region of a patient is acquired. For MR, the magnetic resonance data is k-space data. Fourier analysis is performed to reconstruct the data from the k-space into a three-dimensional object or image space.

The medical image represents tissue and/or bone structure of the patient. Alternatively, the medical image represents flow, velocity, or fluids within the patient. In other embodiments, the medical image represents both flow and structure. For PET and SPECT, the scan data represents function of the tissue, such as uptake.

The medical image represents a one, two, or three-dimensional region of the patient. For example, the medical image represents an area or slice of the patient. Values are provided for each of multiple locations distributed in two or three dimensions. The medical image is acquired as a frame of data. The frame of data represents the scan region at a given time or period. The dataset may represent the area or volume over time, such as providing a 4D representation of the patient.

The scan data is acquired local to the processing of act 16. For example, the medical scanner used to acquire the scan data also implements the part of the machine-learnt classifier. As another example, a workstation or computer connected with a local area network for a hospital or facility implements the part of the machine-learnt classifier in the hospital or facility where the medical scanner is located. Alternatively, the scan data is acquired at a location remote from the implementation of act 16.

In act 14, parameters of the layers of the machine-learnt classifier used locally are stored. The storage is local to the implementation in act 16. The memory for a machine or computer implementing the part of the machine-learnt classifier stores the parameters of that part. The weights or values, such as in a matrix, for the parameters are stored.

The parameters of the part form the layers. For a convolution layer, the parameters may be one or a group of filter kernels applied for that layer. The number of outputs, size of filter kernel, weights, interconnections, or other parameters defining a layer are stored. Any now known or later developed parameterization of layers in a machine-learnt classifier may be used.

The stored parameters are encrypted in one embodiment. Any encryption may be used. A key or password is required to decrypt the values of the parameters for use. If the data output in act 22 is intercepted by a third party, the original medical data of act 12 may then be attempted to be reconstructed by content reconstruction. In order to perform this reconstruction, the attack must have access to the values of the parameters of layers 1-n. Encrypting the design and values of the locally run 1-n layers may prevent the attacker from gaining access to the original values. Even if the attacker both intercepted the data and defeated the encryption on the local application, if n is chosen at a large enough value, an accurate reconstruction of the original image may be impossible. In alternative embodiments, the values of the parameters and/or the design (e.g., which parameters) are not encrypted.

In act 16, just a part of the machine learnt classifier is operated locally. The machine-learnt classifier includes a sequence of operations, each of which is responsive to the training. For example, deep learning is applied to create a multiple layer machine-learnt classifier, such as a neural network. As another example, the machine learning includes learning one or more features to be used by subsequent processing. One part of the machine-trained classifier extracts the features. Other parts may process successive features to determine a class membership. Only part of the overall machine-learnt classifier is applied locally. For example, n of k layers, where k is greater than n, are applied locally, such as at a given location (e.g., building, room, facility, or same computer system).

The local part of the machine-trained classifier is operated on data appropriate for the classification. The classifier is trained to classify from one or more types of input information. For example, data related to a person is input. The data may be a photograph, medical scan data, medical sensor data, and/or other data.

In the medical imaging example, the machine-learnt classifier is trained to provide automated computer aided diagnosis (CAD). The CAD reports identified tumors, segmented tissue, type of lesion, or other diagnostic information. In traditional cases for cloud-based application of machine-learnt classification, scan data for an entire volume is passed through a computer network. The proposed division of the machine-trained classifier may reduce data size as well as protecting privacy. By selecting a greater value of n and/or an activation layer with fewer inputs for local operation, greater compression of the data occurs.

In another example, not falling under the scope of the invention, the machine-learnt classifier is operated for object or person identification from a photograph. The local part of the machine-learnt classifier operates on the photograph. Personal or other photographs may benefit from advanced algorithms to identify and label people and/or locate scenes. However, privacy is a concern if the images are loaded into the cloud for processing, such as not wanting a person to be identified in a way that may be stolen by others. Data security breaches have previously compromised people's personal photos. Using the proposed methods, the user's local computer only sends abstracted data that was specifically designed for the classification application and of little value for alternative uses. This would not only reduce the value of the data obtained from a comprised server, but also make it a less valuable target for hackers.

In yet another example, not falling under the scope of the invention, the machine-learnt classifier is operated for analysis of measurements by sensors from a person, such as wearable devices. Wearable devices or other sensors collect analytical information that may be transmitted for cloud-based analysis. By implementing part of the machine-learnt classification locally, the data may be sent in a compressed format that hides user identifiable information. As an example, a user has a watch that records detailed heart rate data. The data sent to a cloud network is in a compressed format ideal for the intended application. The user may have increased confidence since the data has little resemblance to the original form due to operating part of the classifier locally.

To operate the local part of the machine-learnt classifier, the input data is processed in act 18. In the medical imaging example, the scan data is processed. A processor operates on the scan data, inputting the scan data to a first layer, generating output of the first layer, and then forwarding the output as input to any next layer. This processing continues through the layers of the deep-learnt network. After passing through all of the local layers, the last local layer outputs an abstracted feature set of data to be passed to the cloud for further processing through the rest of the machine-learnt classifier in act 28. Any number of layers may be used locally, such as one or more.

In the example of Figure 2, there are n layers in the subset of layers handled locally. For example, the machine-learnt classifier includes k layers. The design of the layers (e.g., type of layers and number of layers) is created as part of the learning or is set by a programmer. The parameters defining each layer are learned by machine learning. Training data representing scans of other patients with known classifications are used to train the machine-learnt classifier. In this example, the deep-learnt network is a neural network learnt with two sets of layers. The first set of layers 1-n are assigned to be processed locally, and the second set of layers k-n are assigned to be processed in the cloud or remote from the local processing. The local set may be one, two, or more layers (Figure 2 shows at least 3 layers in the local set). The cloud set may be one, two or more layers (Figure 2 shows two layers in the cloud set).

Any division of layers may be provided. A granularity is provided by selecting a value n for the pass off into the cloud-based processing or application. The choice of n determines the degree of abstraction in the data passed. The layers assigned for local processing may be provided to one or more locations, such as to many hospitals. Where the machine-learnt classifier is to be upgraded, the upgrading of the layers processed in the cloud may be more easily managed, such as upgrading at one or a few number of locations. The local layers are essentially fixed as the local layers may not be as easily upgraded as the layers in the cloud. Hence, the number and type of layers to be assigned for local processing may be kept as robust as possible. Limiting the local processing to as few layers as possible may help, but is traded off with the level of anonymization. Using convolution, max pooling or both, without using fully connected or up convolution layers locally may lead to more robust division. Any layers, including fully connected and/or up convolution layers are processed in the cloud. In alternative embodiments, one or more fully connected layers and/or up convolution layers are included locally.

In another approach to keep the local layers robust, training weights for the local layers are initialized with weights obtained from networks trained on millions or billions of images even if these images are not necessary medical images. Training for another classifier or using the same layer structure but different types of images with many more available samples is used to initialize training, with the remaining training being based on the initial weights and the training data specific to the classification for the medical imaging.

In act 20, the output feature set of anonymized data from the processing through the local layers is encrypted. Any encryption may be used, such as lossless encryption. Encryption and decryption may be included in the deep learning where the layer distribution is known prior to training, so that lossy encryption may be used. In other embodiments, the training is performed without encryption. The encryption is provided for implementation. In alternative embodiments, encryption is not used. The abstraction by processing through the local layers is relied upon for privacy without encryption.

In act 22, the processor, using an interface card and communications network, transmits the output feature set to the cloud server or processor. For example, a processor in a facility with the medical scanner, such as being part of the medical scanner, transmits to a cloud server for completing classification with a remainder of the machine-learnt classifier. The cloud server is remote from the facility, such as being in a different building, city, state or country.

Any of various types of information are transmitted. For example, the transmission includes correct results for retaining, selection of an application, and/or the output of the last layer processed locally. Additional, different, or fewer types of information may be transmitted, such as just transmitting the output of the last or other local layers.

In one embodiment, the local layers may be used with more than one other application. The local layers are common to multiple machine-learnt classifiers. The data obtained from the nth activation layer may be placed into a completely different network for detection or performing a similar task. For example, a network is designed to analyze and determine a tumor in the liver as benign or malignant. The same features at the nth layer can also potentially be used to determine if a tumor in the kidney is benign or malignant. This can be done by replacing the n+1 through k layers (i.e., cloud layers) with a new classification network specific for kidney tumors. The different cloud layer sets for the different applications are trained to use the same local layers as initial layers. Different training data is used for a subsequently created application, but is initially processed by the fixed local layers. Unlike the traditional method where a physician or hospital would have to surrender the complete dataset, only the features which are useful for tumor classification or training of the cloud layers are transmitted. This limits the reuse value of the data and establishes a trust that is set by design, allowing for potentially more collaborators and a more secure and potentially compressed transfer of data. For use, the transmission includes a selection of which of the applications and corresponding remainder of the machine-learnt classifiers are to be used in the cloud for a given patient.

The transmission includes an output of the local layers. The output from the last of the local layers is transmitted. For example, the activation data from the last hidden layer of the local set of layers is transmitted. Output from other layers may be used, depending on the design of the machine-learnt classifier.

The output, even if not encrypted, is different from the scan data input for local processing. The processing of the layers creates an output that is more anonymous to the patient than the original patient data. For imaging, the output, if used to generate an image, may not include features that may be easily related to a particular patient. There may be no way to relate the data to a particular patient without reverse engineering or having the parameters or weights for the local layers. There may be no way to relate the data even knowing the parameters or weights of the local layers. The processing of the local layers generates an output that is abstract relative to the scan data, so the processing of the local layers removes identifying information of the person while maintaining information specific to the classification.

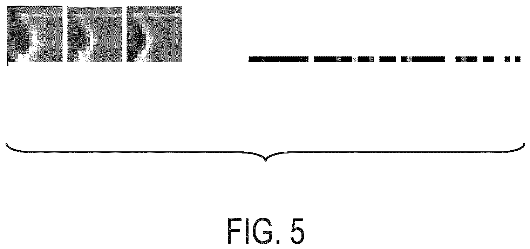

Figure 5 shows an example of anonymized activation data to be transmitted after passing through local layers of a neural network. The machine-learnt classifier classifies regions of the patches or regions in a volume as either bone, vessel, or neither. The images on the left show the original image data while the linear structure on the right represents the same data after passing through a single layer.

The activation data is different than the input scan data. In this example, a volume representing one or more boney regions of a patient are used as the input. The volume is a 15X15X15 sub-volume of a volumetric CT image, providing 15 slices. Figure 5 shows 3 of the 15 slices as images. Once this volume is passed through a single local layer (i.e., before reaching a fully connected layer three in the cloud), the activation data is just a vector of data comprised of features determined from previous layers. This activation data is comprised of floating point values. For viewing, the floating point values are represented as an image by linearly mapping the largest value to white and the lowest value to black. Note that this linear image on the right side of Figure 5 is for illustrative purposes and such a representation may not be performed. Instead, these floating point values are transmitted.

The image shows an example of the anonymization. Even if the floating point values were obtained, the abstracted floating point values do not indicate sufficient structure in the bone of the particular patient to distinguish that patient from others. The partially processed data is unlike and even compressed compared to the original data. The partial network is trained specifically for bone detection, so the altered data may be difficult to use for other purposes. In fact, this data may even be sent unencrypted without compromising the patient's identity since reconstruction is not possible or extremely difficult.

Rather than passing the entire volume to the cloud, the local processing through just part of the deep-learnt network reduces the size of the transmitted data and makes the data less likely to be useful if accessed by an unauthorized entity or even the entity running the application. By selecting a greater value of n or an activation layer with fewer inputs, the activation data to be transmitted is inherently compressed. Alternatively, compression is not provided if the amount of data necessary by the layer in the cloud is greater or equal to that of the previous layer.

For photographs, not falling under the scope of the invention, the activation data transmitted in act 22 is different than the photograph. The local computer only sends abstract data that was specifically designed for a given application and of little value for alternative uses. This not only reduces the value of the data obtained from a comprised server, but also makes the transmitted data a less valuable target for hackers. Any compression performed by the local processing may be beneficial as well.

For sensors, not falling under the scope of the invention, such as wearable devices, the activation data may be different and compressed by the local processing. The activation data is sent in a compressed format that hides user identifiable information. As an example, a user has a watch that records detailed heart rate data. After local processing of just part of the machine-learnt classifier, the activation data is sent to the remaining classifier in the cloud and stored in a compressed format ideal for the intended application. Due to the local processing, the user may have increased confidence in the application provider that the transmitted data has little resemblance to the original form.

The transmission of act 22 provides the activation data to the cloud server or other processor remote from the local processing of act 18. In act 24, the transmission is received by the cloud server or remote processor. The feature data output by the part of the machine-learnt classifier is received. This feature data is more anonymous to the patient due to the processing of the original data by part but not all of the machine-learnt processing.

If the activation data is encrypted, then decryption is applied. The processor decrypts the activation data using a key or pass code. If the activation data transmitted is not encrypted, then decryption is not applied.

In act 26, the processor selects the application. Where more than one application is provided, the application to be applied for a given patient is selected. For example, a liver tumor application is selected, and a kidney tumor application is not selected. Both applications use the same features from the local processing to classify, but for different types of tissue. Each application corresponds to a different remaining part of the machine-learnt classifier. For example, the liver tumor application has different parameters, weights, and/or layers in the layers n+1 through k than the breast tumor application. The selection determines which of the remaining parts of the machine-trained classifiers to use.

In act 28, a machine (e.g., cloud server or remote processor) performs a remainder of the machine-learnt processing. The received feature or activation data is input to the remaining part of the machine-learnt classifier. The output of the local layers is input to a first of the cloud layers of the deep-learnt network. For example, the output of layer n of Figure 2 is input to layer n+1 (k-1 of Figure 2). Where the remaining part includes two or more layers, the outputs from each of the layers are used as inputs to the subsequent layer. The classification is performed with the multiple layers, such as fully connected, up convolution, convolution, and/or max pooling layers.

Based on the training, the remaining part of the machine-learnt classifier outputs a classification. For example, the last layer outputs the classification. The classification may be binary, such as benign or malignant. More than two classes may be used. The classification may be probabilistic, such as a probability of being in each of two or more classes. In other embodiments, the classification is of a type of tissue, membership in a group, prediction of outcome, identification of anatomy, identification of a lesion, stage of disease, or other classification. Additionally, a segmentation or evaluation can also be output. The scan data originally input to the local part of the classifier is completely classified by the remote or cloud part of the classifier.

In act 30, the machine transmits the results of the machine-learnt processing back to the local machine. For example, a cloud server or remote processor transmits the classification of the scan data for the patient over the communications network to a local processor, such as a processor of a workstation or medical scanner at a facility caring for the patient. The transmission returns the results to the local part of the machine-learnt classifier. The results are provided in response to receipt of the feature data. In other embodiments, the results are stored and looked up by an authorized user, so the transmission may not occur until a later request. In alternative embodiments, the result or classification is transmitted to another location, such as a computer associated with a department, medical record system, or physician. This transmission is instead of or in addition to transmission back to the processor applying the local part of the classifier.

In act 32, the result from the remote processor or cloud server is received by the local processor. For example, the output of the part of the neural network applied by the cloud server is received by the processor that performed the local part of the neural network. The classification, evaluation, or segmentation of the medical imaging data is received. For another embodiment, not falling under the scope of the claims, the identification of a person or labeling of content of a photograph is received. In another embodiment, analysis of measurements for a person or object are received. In any of the embodiments, the output from the operation of the remaining part (e.g., cloud part) of the neural network or another machine-learnt classifier is received. The output is based on the input to the initial part and processing of the initial part. Where multiple possible applications are provided for a given feature set, the output received is for the selected application.

In act 34, the output is displayed. Any display may be provided, such as displaying a medical record with a classification result. The display may be part of a notice or pop-up. For imaging, the display may be an annotation, marking, coloring, highlighting, or marking on or by a display of an image from the scan data. The display may provide an output for computer assisted diagnosis, such as for a second review of medical imaging results to assist a physician.

For cloud-based applications where privacy of the data is important, using division of the machine-learnt classifier between local and remote processing may keep end-user trust. The data created by partially processing through the machine-learnt classifier is more anonymous than scan data and has use limited to the application, so avoids the risks of providing scan data or other original data to the cloud server. As a specific example in the case of medical data, big data usage and cloud applications are becoming more pervasive. The data to develop and maintain such applications is becoming more valuable to the point where companies are being bought just for their data. By using division of the machine-learnt classifier between local and cloud machines, data may be safely shared with a remote service provider. This may increase the ability of the service provider to access a greater amount of useful information even if the original data is not accessible. Data unobtainable due to cost or other restrictions may now be available due to trust insured by design rather than trust ensured by a pledge to protect.

Access to more information or a greater number of samples may assist in retraining, providing more effective classification. In act 22, the correct label for a given case is transmitted to the cloud server, remote processor, or other processor used for retraining. The correct label is a ground truth or physician reviewed result. For example, the physician views images from the medical scan data, biopsies the lesion, or otherwise verifies the classification. The physician verified result is transmitted.

The verified result is transmitted with the feature data output by partial operation of the machine-learnt classifier. The verified results may be provided for retraining alone so that the remainder of the classification is not applied. Alternatively, the verified results are provided based on the complete classification. A physician for the patient or other physician reviews the output of the machine-learnt classifier provided in act 30. This classification is verified as correct or incorrect. In the cases of incorrect results, if the user is willing to supply the correct values, the correct classification along with the feature data sent in act 22 may be used to retrain the n+1 to k layers of the network.

In act 36, the machine-learnt classifier is retrained. The retraining may be incremental, such as retraining for each verified result or group of verified results. The feedback of the verified result of incorrect classification is used to adjust one or more weights. The adjustment or retraining may be limited to avoid over adjustment. Alternatively, the retraining may be a full retraining, such as using a collection of verified results and corresponding activation data with the original training data to retrain from scratch. To avoid complications in changing the portion of the classifier at one or more local locations, the local portion is not retrained. For example, layers 1-n of Figure 2 are maintained the same. The retraining is just for the cloud or remote layers of the machine-learnt classifier. For example, layers n+1 through k are retrained. The retraining uses the output from the local layers and the ground truth (verified results) to retrain the remainder of the deep-learnt network. Alternatively, the entire machine-learnt classifier is retrained. The retrained local layers are provided to the local machines for operation. In such a situation, a version number may be supplied with the data, and the cloud-based classifier refers to older or newer versions of the network to remain compatible with older local clients.

## Claims

1. A method for use of machine-learnt classifier in medical imaging, the method comprising:
acquiring, with a medical scanner, scan data representing a patient;
processing, with a first processor, the scan data through a first set of layers of a deep-learnt network, the deep-learnt network comprising the first set of layers and a second set of layers, the first set of layers comprising two or more layers;
transmitting an output of the first set of layers over a communications network from the first processor to a second processor, the output being different than the scan data and being more anonymous to the patient than the scan data;
processing, with the second processor, the output of the first set of layers through the second set of layers of the deep-learnt network, an output of the second set of layers being a classification of the scan data; and
transmitting the classification of the scan data for the patient over the communications network from the second processor to the first processor,
**characterized in that**
the method further comprises
transmitting a physician verified result of the classification to the second processor; and
retraining the second set of layers of the deep learnt network based on the physician verified result and the output of the first set of layers and without retraining the first set of layers.

2. The method according to claim 1, wherein acquiring scan data comprises acquiring the scan data as computed tomography, magnetic resonance, ultrasound, positron emission tomography, or single photon emission computed tomography data.

3. The method according to claim 1 or 2, wherein processing the scan data comprises processing with the deep-learnt network being a neural network learnt from training data of scans of other patients with known classifications,
and/or
wherein processing the scan data comprises processing with the first set of layers comprising a convolutional layer, a max pooling, or both the convolutional layer and the max pooling layers; and
wherein processing the output comprises processing with the second set of layers comprising a fully connected layer, an up convolutional layer, or both the fully connected layer and the up convolutional layer.

4. The method according to any of the preceding claims, wherein processing the scan data comprises inputting the scan data to a first layer of the first set of layers, and inputting an output of the first layer to a second layer of the first set of layers.

5. The method according to any of the preceding claims, wherein transmitting the output comprises transmitting the output as abstracted data relative to the scan data, and wherein transmitting the classification comprises transmitting the classification as identification of anatomy, identification of a lesion, identification as benign or malignant, or staging.

6. The method according to any of the preceding claims, wherein transmitting the output comprises transmitting from the first processor in a facility with the medical scanner to the second processor, the second processor comprising a cloud server.

7. The method according to any of the preceding claims, wherein processing the output comprises processing with two or more layers in the second set of layers.

8. The method according to any of the preceding claims, further comprising:
storing parameters of the first set of layers as encrypted,
and/or
further comprising:
encrypting the output, wherein transmitting the output comprises transmitting the output as encrypted; and
decrypting the output as encrypted by the second processor prior to processing the output.

9. Non-transitory computer readable storage medium storing data representing instructions executable by a processor, wherein the data, when executed by a processor, causes the processor to implement the method according to any of the claims 1 to 8.

## Patentansprüche

1. Verfahren zur Verwendung eines maschinell erlernten Klassifizierers in der medizinischen Bildgebung, wobei das Verfahren umfasst:
Erfassen von Scandaten eines Patienten mit einem medizinischen Scanner;
Verarbeiten der Scandaten mit einem ersten Prozessor durch einen ersten Satz von Schichten eines Deep-Learn-Netzes, wobei das Deep-Learn-Netz den ersten Satz von Schichten und einen zweiten Satz von Schichten umfasst, wobei der erste Satz von Schichten zwei oder mehr Schichten umfasst;
Übertragen einer Ausgabe des ersten Satzes von Schichten über ein Kommunikationsnetz von dem ersten Prozessor zu einem zweiten Prozessor, wobei sich die Ausgabe von den Scandaten unterscheidet und für den Patienten anonymer ist als die Scandaten;
Verarbeiten der Ausgabe des ersten Satzes von Schichten mit dem zweiten Prozessor durch den zweiten Satz von Schichten des Deep-Learn-Netzes, wobei eine Ausgabe des zweiten Satzes von Schichten eine Klassifizierung der Scandaten ist; und
Übertragen der Klassifizierung der Scandaten für den Patienten über das Kommunikationsnetz von dem zweiten Prozessor an den ersten Prozessor, **dadurch gekennzeichnet, dass** das Verfahren ferner umfasst Übermitteln eines ärztlich verifizierten Ergebnisses der Klassifizierung an den zweiten Prozessor; und
Umlernen des zweiten Satzes von Schichten des Deep-Learn-Netzes auf der Grundlage des vom Arzt verifizierten Ergebnisses und der Ausgabe des ersten Satzes von Schichten und ohne Umlernen des ersten Satzes von Schichten.

2. Verfahren nach Anspruch 1, wobei das Erfassen von Scandaten das Erfassen der Scandatenals Computertomographie-, Magnetresonanz-, Ultraschall-, Positronen-Emissions-Tomographie- oder Einzelphotonen-Emissions-Computertomographiedaten umfasst.

3. Verfahren nach Anspruch 1 oder 2, wobei die Verarbeitung der Scandaten die Verarbeitung mit dem Deep-Learn-Netz umfasst, bei dem es sich um ein neuronales Netz handelt, das aus Trainingsdaten von Scans anderer Patienten mit bekannten Klassifizierungen gelernt wurde,
und/oder
wobei die Verarbeitung der Scandaten die Verarbeitung mit dem ersten Satz von Schichten umfasst, der eine Faltungsschicht, eine Max-Pooling-Schicht oder sowohl die Faltungsschicht als auch die Max-Pooling-Schicht umfasst; und
wobei die Verarbeitung der Ausgabe die Verarbeitung mit dem zweiten Satz von Schichten umfasst, der eine vollständig verbundene Schicht, eine Aufwärtsfaltungsschicht oder sowohl die vollständig verbundene Schicht als auch die Aufwärtsfaltungsschicht umfasst.

4. Verfahren nach einem der vorhergehenden Ansprüche, wobei die Verarbeitung der Scandaten die Eingabe der Scandaten in eine erste Schicht des ersten Satzes von Schichten und die Eingabe einer Ausgabe der ersten Schicht in eine zweite Schicht des ersten Satzes von Schichten umfasst.

5. Verfahren nach einem der vorhergehenden Ansprüche, wobei das Übertragen der Ausgabe das Übertragen der Ausgabe als abstrahierte Daten in Bezug auf die Scandaten umfasst und wobei das Übertragen der Klassifizierung das Übertragen der Klassifizierung als Identifizierung der Anatomie, Identifizierung einer Läsion, Identifizierung als gutartig oder bösartig oder Staging umfasst.

6. Verfahren nach einem der vorhergehenden Ansprüche, wobei die Übertragung der Ausgabe die Übertragung vom ersten Prozessor in einer Einrichtung mit dem medizinischen Scanner zum zweiten Prozessor umfasst, wobei der zweite Prozessor einen Cloud-Server umfasst.

7. Verfahren nach einem der vorhergehenden Ansprüche, wobei die Verarbeitung der Ausgabe die Verarbeitung mit zwei oder mehr Schichten im zweiten Satz von Schichten umfasst.

8. Das Verfahren nach einem der vorangegangenen Ansprüche , das ferner umfasst:
Speichern der Parameter des ersten Satzes von Schichten in verschlüsselter Form,
und/oder
weiter umfassend:
Verschlüsseln der Ausgabe, wobei das Übertragen der Ausgabe das Übertragen der Ausgabe als verschlüsselt umfasst; und
Entschlüsseln der verschlüsselten Ausgabe durch den zweiten Prozessor vor der Verarbeitung der Ausgabe.

9. Nicht-transitorisches computerlesbares Speichermedium, das Daten speichert, die von einem Prozessor ausführbare Befehle darstellen, wobei die Daten, wenn sie von einem Prozessor ausgeführt werden, den Prozessor veranlassen, das Verfahren nach einem der Ansprüche 1 bis 8 durchzuführen.

## Revendications

1. Méthode d'utilisation d'un classificateur à apprentissage automatique dans le domaine de l'imagerie médicale :
acquérir, à l'aide d'un scanner médical, des données de balayage représentant un patient ;
traiter, à l'aide d'un premier processeur, les données de balayage à travers un premier ensemble de couches d'un réseau à apprentissage profond, le réseau à apprentissage profond comprenant le premier ensemble de couches et un deuxième ensemble de couches, le premier ensemble de couches comprenant deux couches ou plus ;
transmettre une sortie du premier ensemble de couches sur un réseau de communication du premier processeur à un second processeur, la sortie étant différente des données de balayage et plus anonyme pour le patient que les données de balayage ;
traiter, avec le second processeur, la sortie du premier ensemble de couches à travers le second ensemble de couches du réseau à apprentissage profond, une sortie du second ensemble de couches étant une classification des données de balayage ; et
transmettre la classification des données de balayage du patient sur le réseau de communication du second processeur au premier processeur, **caractérisé en ce que** le procédé comprend en outre
transmettre au second processeur le résultat de la classification vérifié par le médecin ; et
réentraîner le deuxième ensemble de couches du réseau d'apprentissage profond sur la base du résultat vérifié par le médecin et de la sortie du premier ensemble de couches, sans réentraîner le premier ensemble de couches.

2. Méthode selon la revendication 1, dans laquelle l'acquisition de données de balayage comprend l'acquisition de données de balayage sous forme de tomographie informatisée, de résonance magnétique, d'échographie, de tomographie par émission de positrons ou de tomographie par émission d'un seul photon.

3. Méthode selon la revendication 1 ou 2, dans laquelle le traitement des données de balayage comprend le traitement avec le réseau à apprentissage profond qui est un réseau neuronal appris à partir de données d'entraînement de scanners d'autres patients avec des classifications connues,
et/ou
dans lequel le traitement des données de balayage comprend le traitement avec le premier ensemble de couches comprenant une couche convolutive, une mise en commun maximale, ou à la fois la couche convolutive et les couches de mise en commun maximale ; et
dans lequel le traitement de la sortie comprend le traitement avec le deuxième ensemble de couches comprenant une couche entièrement connectée, une couche convolutionnelle ascendante, ou à la fois la couche entièrement connectée et la couche convolutionnelle ascendante.

4. Méthode selon l'une des revendications précédentes, dans laquelle le traitement des données de balayage comprend l'entrée des données de balayage dans une première couche du premier ensemble de couches, et l'entrée d'une sortie de la première couche dans une deuxième couche du premier ensemble de couches.

5. Méthode selon l'une des revendications précédentes, dans laquelle la transmission de la sortie comprend la transmission de la sortie sous forme de données abstraites relatives aux données de balayage, et dans laquelle la transmission de la classification comprend la transmission de la classification sous forme d'identification de l'anatomie, d'identification d'une lésion, d'identification comme bénigne ou maligne, ou de stadification.

6. Méthode selon l'une des revendications précédentes, dans laquelle la transmission de la sortie comprend la transmission du premier processeur dans une installation avec le scanner médical au deuxième processeur, le deuxième processeur comprenant un serveur en nuage.

7. Méthode selon l'une des revendications précédentes, dans laquelle le traitement de la sortie comprend le traitement de deux couches ou plus dans le deuxième ensemble de couches.

8. Procédé selon l'une quelconque des revendications précédentes , comprenant en outre :
stocker les paramètres de la première série de couches sous forme cryptée,
et/ou
comprenant en outre
crypter la sortie, la transmission de la sortie comprenant la transmission de la sortie sous forme cryptée ; et
décryptage de la sortie chiffrée par le second processeur avant le traitement de la sortie.

9. Support de stockage non transitoire lisible par ordinateur stockant des données représentant des instructions exécutables par un processeur, dans lequel les données, lorsqu'elles sont exécutées par un processeur, amènent le processeur à mettre en oeuvre la méthode selon l'une des revendications 1 à 8.
